# FASCICULE DE BREVET EUROPEEN

(11) **EP 3 055 227 B1**
(45) Date de publication et mention de la délivrance du brevet: **01.08.2018**
(21) Numéro de dépôt: 14786824.4
(22) Date de dépôt: 08.10.2014
(51) Int. Cl.: B65D 83/00, B65D 47/20

(54) **DISPOSITIF DE DISTRIBUTION ET DE PROTECTION DE FLUIDE COMPORTANT UN OBTURATEUR À FENTE**
VORRICHTUNG ZUR ABGABE UND ZUM SCHUTZ EINER FLÜSSIGKEIT MIT EINEM GESCHLITZTEN STOPFEN
DEVICE FOR DISPENSING AND PROTECTING A FLUID, COMPRISING A SLIT STOPPER

(30) Priorité: 10.10.2013 FR 1359844
(43) Date de publication de la demande: 17.08.2016
(73) Titulaire: Courtin, Karine, 92100 boulougne Billancourt (FR)
(72) Inventeur: Courtin, Karine, 92100 boulougne Billancourt (FR)
(74) Mandataire: Brevalex
(86) Numéro de dépôt international: PCT/EP2014/071500
(87) Numéro de publication internationale: WO 2015/052211

(56) Documents cités:
- FR-A- 955 907
- US-A- 2 620 949
- US-A- 5 186 368
- US-A- 6 145 707
- US-A1- 2008 035 677
- US-B1- 6 450 375

## Description

### DOMAINE TECHNIQUE

L'invention concerne un dispositif de distribution de fluide qui a pour but de limiter les risques de pollution de la préparation à distribuer par de l'air ou par des contaminants bactériens notamment.

L'invention concerne par exemple un dispositif de distribution d'un produit se présentant sous la forme d'une crème, d'une pâte, d'un gel ou d'un liquide pour l'industrie pharmaceutique, cosmétique, chimique ou alimentaire, qui est contenu dans un tube semi-rigide ou un pot.

### ÉTAT DE LA TECHNIQUE ANTÉRIEURE

De nombreux produits sous forme pâteuse ou liquide sont contenus dans un récipient qui permet à la fois la conservation du produit et la distribution de celui-ci.

La conservation du produit est généralement assurée grâce à un récipient hermétique à l'air et aussi en introduisant dans le produit des éléments conservateurs, antioxydants, bactéricides etc.

Une source de pollution du produit est aussi située au niveau du dispositif de distribution. En effet, le dispositif de distribution comporte une ouverture de sortie du produit au niveau de laquelle de l'air peut pénétrer à l'intérieur du contenant et polluer le produit.

Aussi, le prélèvement du produit s'effectue en balayant la surface externe du dispositif, avec le doigt par exemple. Cette action de balayage apporte des bactéries ou autres éléments polluants sur la surface externe, mais aussi dissémine ceux-ci.

Le document WO-A-2013/039482 décrit un obturateur à fente qui permet d'obturer complètement l'ouverture du dispositif de distribution, empêchant ainsi à l'air de pénétrer dans le contenant.

L'obturateur à fente consiste en un disque en matériau déformable élastiquement, au centre duquel une fente, par exemple en forme de croix, est réalisée.

Lorsqu'une pression est exercée sur le contenant, la pression du produit à distribuer augmente, provoquant alors la déformation de l'obturateur, au niveau de la fente. Les parties élastiques formées par la fente se déforment élastiquement selon un mouvement axial vers l'avant et radial vers l'extérieur, en suivant le mouvement du produit.

Lorsque la pression sur le contenant est relâchée, le produit n'appuie pas suffisamment sur l'obturateur, qui reprend alors sa position initiale. Lors de ce retour élastique de l'obturateur vers sa forme initiale, une certaine quantité de produit, qui a été en contact avec l'air extérieur et divers éléments pouvant polluer le produit, retourne à l'intérieur du contenant.

Il n'y a donc pas une protection complète du produit par un tel obturateur.

Un obturateur de l'art antérieur, et présentant les caractéristiques du préambule de la revendication 1 annexée, est décrit dans FR 955907.

L'invention a pour but de proposer un dispositif de distribution comportant un obturateur à fente qui est réalisé de manière à protéger complètement le produit contenu dans le récipient contre l'intrusion d'air et d'éléments polluants, afin de garantir qu'à une utilisation prochaine du produit, celui-ci ne sera pas altéré.

### EXPOSÉ DE L'INVENTION

L'invention propose un dispositif de distribution d'un produit fluide conforme à la revendication 1. Une telle réalisation du dispositif de distribution permet, par le mouvement relatif des deux parois formées par la fente, lors de l'ouverture et de la fermeture de l'orifice de distribution, d'empêcher qu'une portion du produit qui aurait pu être en contact avec un élément extérieur, ne revienne à l'intérieur du contenant. La rainure formée sur la face arrière de l'obturateur à fente permet de favoriser l'écartement des deux parois, sous la pression exercée par le produit. L'obturateur à fente comporte une nervure en saillie par rapport à sa paroi périphérique qui est reçue dans une gorge associé du logement central, et en ce qu'un jeu radial est présent entre le fond de la gorge radiale et l'extrémité libre de la nervure.

De préférence, le corps comporte une face externe recouverte par une couche en un matériau bactéricide ou au moins bactériostatique et la couche de matériau comporte une ouverture centrale traversée par l'obturateur à fente de manière telle que la face externe de l'obturateur à fente affleure avec la face externe de la couche de matériau.

De préférence, la couche de matériau est réalisée par dépôt métallique sur la face externe du corps.

De préférence, la couche de matériau consiste en une plaque métallique sertie sur le corps, en recouvrant la face externe du corps.

De préférence, la couche de matériau est réalisée à partir de cuivre ou d'argent.

De préférence, la face externe de la couche de matériau et la face externe de l'obturateur à fente forment une surface d'application plane, concave ou convexe.

De préférence, le dispositif est réalisé d'une seule pièce avec un récipient contenant le produit.

De préférence le récipient est rigide et comporte une paroi mobile et une paroi fixe qui porte le dispositif.

De préférence, le récipient est rigide et comporte une paroi mobile qui porte le dispositif.

### BRÈVE DESCRIPTION DES DESSINS

D'autres caractéristiques et avantages de l'invention apparaîtront à la lecture de la description détaillée qui suit pour la compréhension de laquelle on se reportera aux figures annexées parmi lesquelles :
- la figure 1 est une représentation schématique en section axiale d'un dispositif selon l'invention ;
- la figure 2 est une vue similaire à celle de la figure 1, dans laquelle les parois de la fente sont écartées lors de la distribution du produit ;
- la figure 3 est une vue similaire à celle de la figure 1, dans laquelle la couche métallique est formée par une plaque sertie sur le corps du dispositif ;
- la figure 4 est une vue similaire à celle de la figure 3, dans laquelle le dispositif consiste en un élément rapporté sur le récipient ;
- les figures 5A, 5B, 5C sont des représentations schématiques montrant différentes formes possibles de l'obturateur à fente ;
- les figures 6A et 6B sont des vues similaires à celle de la figure 1, dans lesquelles la surface de distribution du dispositif est courbe et convexe ou concave ;
- les figures 7A et 7B sont des sections de récipients rigides comportant une paroi rigide mobile et dans lesquelles le dispositif est monté ou non sur la paroi mobile.

### EXPOSÉ DÉTAILLÉ DE MODES DE RÉALISATION PARTICULIERS

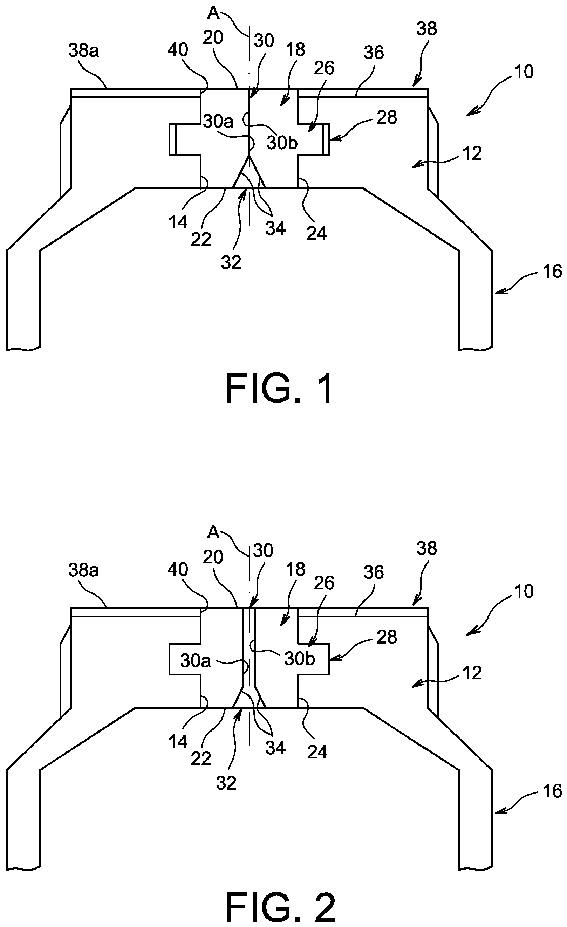

On a représenté aux figures 1 et 2 un dispositif 10 de distribution d'un fluide tel que par exemple une pâte, une crème, un gel ou une solution liquide.

Le dispositif 10 comporte un plan principal A médian situé en son centre.

Le dispositif 10 comporte un corps principal 12 qui est d'orientation principale perpendiculaire au plan principal A et qui comporte un logement central 14.

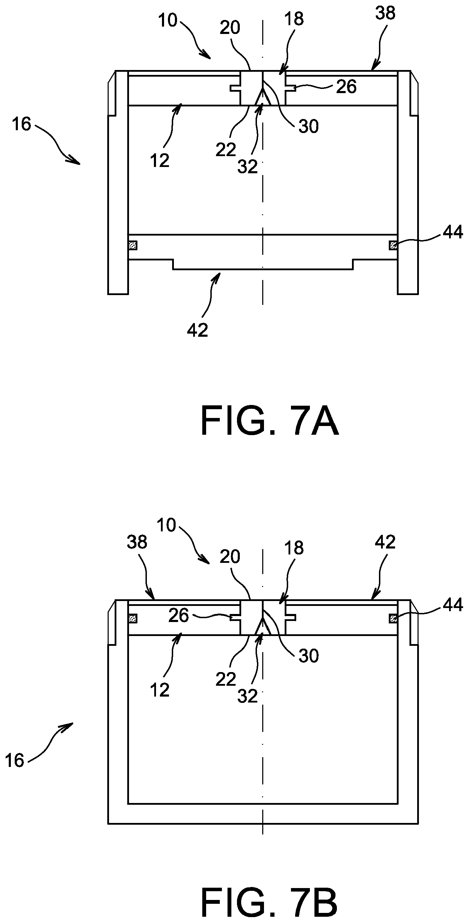

Le dispositif 10 fait partie d'un récipient 16 à l'intérieur duquel le produit à distribuer est contenu. Le récipient consiste par exemple en un tube réalisé en un matériau élastique, semi-rigide, ou mou, ou bien le récipient 16 consiste en un récipient rigide tel qu'un pot, que l'on a représenté à titre d'exemple aux figures 7A et 7B.

Un obturateur à fente 18 est agencé à l'intérieur du logement 14 et ferme de manière hermétique le logement 14.

L'obturateur à fente 18 consiste en un élément déformable élastiquement qui est monté dans le logement 14. Il est délimité par une face externe 20, au niveau de laquelle le produit sort du récipient 16, une face interne 22 qui est en contact avec le produit contenu dans le récipient 16 et une paroi périphérique 24 qui est en contact étanche avec la paroi du logement 14.

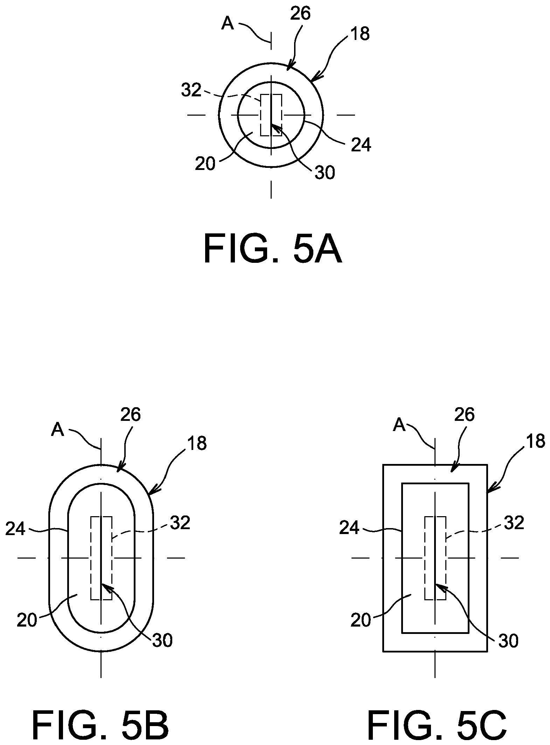

La paroi périphérique 24 comporte une nervure 26, qui est reçue dans une gorge associée 28 réalisée dans le logement 14. La nervure 26 fait saillie par rapport à la paroi périphérique 24 et elle peut s'étendre sur toute la périphérie de celle-ci, comme on peut le voir par exemple aux figures 5A à 5C, ou seulement une partie de celle-ci. La nervure 26 et la gorge 28 associée permettent la rétention de l'obturateur à fente 18 dans le logement 14 par emboitement élastique.

L'obturateur à fente 18 comporte aussi une fente 30 qui s'étend parallèlement au plan principal A et de préférence dans ce plan principal A depuis la face interne 22 jusqu'à la face externe 20. La fente 30 comporte deux parois 30a, 30b en vis-à-vis qui sont accolées l'une contre l'autre lorsque l'obturateur 18 est dans une position de fermeture représentée à la figure 1 et qui sont écartées l'une de l'autre lorsque l'obturateur 18 est dans une position d'ouverture représentée à la figure 2, permettant le passage du produit au travers de la fente 30.

L'ouverture de la fente 30 est obtenue sous l'action de la pression du produit contenu dans le récipient 16. Cette pression, lorsqu'elle devient suffisamment importante, provoque l'écartement des deux parois 30a, 30b en vis-à-vis. Lorsque la pression diminue, les parois 30a, 30b en vis-à-vis se rapprochent l'une de l'autre, jusqu'à venir en appui l'une contre l'autre.

Le déplacement relatif des parois 30a, 30b en vis-à-vis consiste ici en un déplacement des parois selon une même direction orthogonale au plan principal A du dispositif 10 et selon des sens opposés. Ce déplacement relatif est obtenu en réalisant un obturateur à fente 18 dont la distance entre les faces intérieure 22 et extérieure 20 est suffisamment importante par rapport à la dimension de l'obturateur à fente 18 selon une orientation perpendiculaire au plan principal A.

Un tel déplacement relatif des parois 30a, 30b permet, lorsque la pression du produit contenu dans le récipient 16 est relâchée, de comprimer le film de produit présent entre les parois 30a, 30b. Le produit est alors expulsé vers l'intérieur du récipient 16 au travers de la face interne 22 et aussi vers l'extérieur au travers de la face externe 20.

Le produit qui a pu être en contact avec des éléments polluants ou avec de l'air, est ainsi expulsé vers l'extérieur, il ne peut alors pas contaminer le reste du produit, qui retourne à l'intérieur du récipient 16.

Lors du déplacement relatif des parois 30a, 30b en vis-à-vis, l'obturateur à fente 18 se déforme élastiquement. Pour cela il est réalisé en un matériau déformable élastiquement, tel que par exemple en élastomère, en caoutchouc naturel ou synthétique ou en polytétrafluoroéthylène (connu sous la marque Téflon®).

Un jeu est présent entre le fond de la gorge 28 et l'extrémité libre de la nervure 26 associée. Ce jeu diminue lors de la déformation élastique de l'obturateur à fente et de l'écartement des parois 30a, 30b.

Pour favoriser l'écartement des parois sous l'action de la pression du produit, l'obturateur à fente 18 comporte une rainure 32 réalisée dans la face interne 22, qui prolonge la fente 30 vers l'intérieur. La rainure 32 est ici à section en "V", elle comporte ainsi deux faces 34 qui sont inclinées par rapport au plan principal A, dont chacune prolonge vers l'intérieur une paroi 30a, 30b de la fente 30.

Grâce à cette rainure 32, la pression exercée par le produit sur chacune des faces inclinées 34 de la rainure 32 a pour résultante un effort incliné par rapport à la face interne et s'éloignant du plan principal A du dispositif 10.

Le récipient 16 est de préférence réalisé en un matériau déformable élastiquement. Ainsi, pour distribuer une certaine quantité de produit, l'utilisateur exerce une pression sur les parois du récipient 16, provoquant l'augmentation de la pression du produit dans celui-ci, jusqu'à ce que les parois 30a, 30b en vis-à-vis s'écartent l'une de l'autre, l'orifice de distribution du produit est ouvert, permettant la sortie du produit.

Lorsqu'une certaine quantité de produit a été distribuée, l'utilisateur relâche sa pression sur les parois du récipient 16. La pression du produit dans le récipient diminue, les parois 30a, 30b de la fente 30 reviennent élastiquement vers leur position initiale d'appui l'une contre l'autre, refermant ainsi l'orifice de distribution.

L'appui des parois 30a, 30b en vis-à-vis permet à lui seul d'avoir une fermeture de l'orifice de distribution qui est étanche à l'air et aux éléments polluants. Aussi, le récipient 16, qui a été comprimé pour distribuer le produit, a tendance à revenir élastiquement vers sa forme initiale, produisant une certaine aspiration. Cette aspiration résulte de la diminution de la quantité de produit présente dans le récipient 16, à cause de la quantité de produit qui a été distribuée, ce qui empêche au récipient 16 de reprendre sa forme initiale.

L'aspiration force encore plus les parois 30a, 30b de la fente 30 à s'appuyer l'une contre l'autre, pour améliorer encore plus la fermeture de l'orifice de distribution du produit. Et empêcher l'air de rentrer dans le récipient 16.

Afin de réaliser une distribution hygiénique du produit, le dispositif 10 est réalisé de manière à limiter la prolifération de bactéries au niveau de sa face externe de distribution du produit.

Cet objectif est réalisé par la face externe 20 de l'obturateur à fente 18, qui est lisse, c'est-à-dire avec une faible rugosité. Ainsi, elle ne comporte pas d'aspérités dans lesquelles le produit peut être retenu et dans lesquelles les bactéries se développeraient.

Cet objectif est aussi réalisé par l'utilisation d'un matériau ayant un effet bactéricide, ou ayant au moins un effet bactériostatique, c'est-à-dire un matériau limitant le développement des bactéries. Un tel matériau est par exemple du cuivre, de l'argent ou un alliage à base d'un de ces matériaux.

Ce matériau est agencé sur le dispositif 10, en recouvrant la face externe 36 du corps 12 et en formant une couche 38 du matériau.

Selon le mode de réalisation représenté aux figures 1 et 2, la couche 38 de matériau est déposée sur la face d'application 36 par exemple par électrolyse, ou plus particulièrement par galvanoplastie.

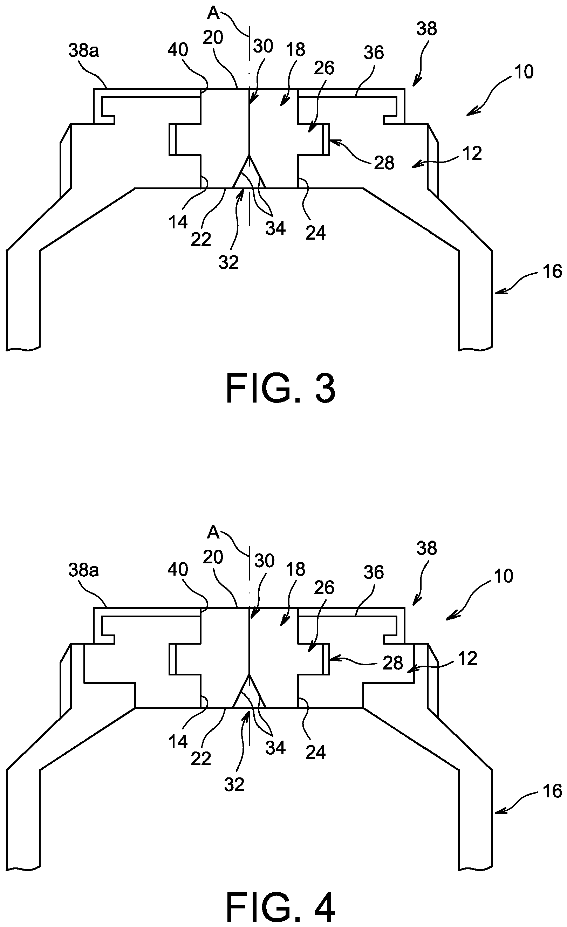

Selon un autre mode de réalisation représenté aux figures 3 et 4, la couche 38 de matériau se présente sous la forme d'une plaque mince, qui est montée sur le corps 12 par sertissage.

La couche de matériau 38, que ce soit la couche déposée ou la plaque mince, comporte un orifice central 40 qui est traversé par l'extrémité externe de l'obturateur à fente 18.

Comme représenté aux figures 5A à 5C, l'obturateur à fente 18, et par conséquent l'orifice central 40 de la couche de matériau 38, peuvent avoir différentes formes, qui sont complémentaires.

Ainsi, par exemple, l'obturateur à fente 18 et l'orifice central 40 sont de forme principale circulaires comme représenté à la figure 5A, ovales ou oblongs, comme représenté à la figure 5B ou bien rectangulaires comme représenté à la figure 5C.

La face externe 20 de l'obturateur à fente 18 affleure avec la face externe 38a de la couche de matériau 38, pour limiter les aspérités au niveau de la liaison entre les deux faces externes 20, 38a, dans lesquelles du produit pourrait être retenu et des bactéries pourraient se développer.

Selon le mode de réalisation représenté aux figures 1 à 3, le dispositif de distribution 10 est réalisé d'une seule pièce avec le reste du récipient 16.

Selon le mode de réalisation représenté à la figure 4, le dispositif de distribution 10 consiste en un élément rapporté sur le reste du récipient 16, qui est solidarisé par sertissage, collage ou soudage par exemple.

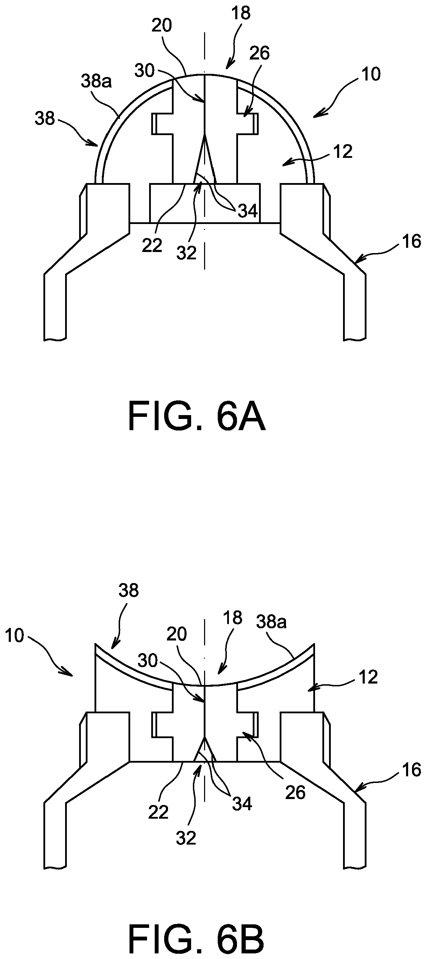

Selon les modes de réalisation représentés aux figures 1 à 4, la face d'application du dispositif de distribution 10, formée par la face externe 20 de l'obturateur à fente 18 et la face externe 38a de la couche de matériau 38, est plane. Il sera compris que l'invention n'est pas limitée à ce mode de réalisation, et qu'elle peut être de forme convexe comme représenté à la figure 6A, ou bien concave comme représenté à la figure 6B.

On a représenté aux figures 7A et7B encore une autre variante de réalisation du dispositif de distribution 10 qui est utilisé en association avec un récipient 16 rigide.

Selon ces modes de réalisation, le récipient 16 comporte une paroi 42 qui est montée coulissante, pour provoquer la sortie du produit, lorsqu'un utilisateur exerce une pression sur cette paroi 42. Lorsque le produit a été distribué, l'utilisateur cesse son action sur la paroi 42, l'obturateur à fente 18 referme automatiquement l'orifice de distribution, comme on l'a exposé ci-dessus, ce qui permet de retenir la paroi coulissante 42 dans sa position.

Des moyens d'étanchéité 44 sont en outre prévus entre la paroi mobile 42 et le reste du récipient 16.

Selon le mode de réalisation représenté à la figure 7A, la paroi mobile 42 est une paroi de fond du récipient 16, qui est opposée à la paroi du récipient comportant le dispositif de distribution selon l'invention.

Selon le mode de réalisation représenté à la figure 7B, la paroi mobile 42 est la paroi supérieure du récipient 16, dans laquelle le dispositif de distribution 18 est monté.

## Revendications

1. Dispositif (10) de distribution d'un produit fluide comportant un corps (12) s'étendant dans un plan orthogonal à un plan principal médian (A) du dispositif (10), qui comporte un logement (14) central,
un obturateur à fente (18) agencé dans le logement (14) central du corps (12), qui est réalisé en un matériau déformable élastiquement et qui est délimité par une face externe (20), une face interne (22) et une paroi périphérique (24),
l'obturateur à fente (18) comportant une fente (30) sensiblement parallèle au plan principal médian (A) du dispositif (10), qui s'étend depuis la face interne (22) jusqu'à la face externe (20), et qui comporte deux parois (30a, 30b) en vis-à-vis qui sont en appui l'une contre l'autre,
l'obturateur à fente (18) comportant une rainure (32) réalisée dans la face interne (22), qui comporte deux faces (34) inclinées dont chacune prolonge vers l'intérieur une paroi (30a, 30b) de la fente (30),
**caractérisé en ce que** les deux parois (30a, 30b) en vis-à-vis sont aptes à s'écarter par un déplacement perpendiculaire au plan principal médian (A) du dispositif (10) sous l'action de la pression du produit à distribuer, par déformation élastique de l'obturateur à fente (18),
et **en ce que** l'obturateur à fente (18) comporte une nervure (26) en saillie par rapport à sa paroi périphérique (24) qui est reçue dans une gorge (28) associé du logement (14) central, et **en ce qu'**un jeu radial est présent entre le fond de la gorge (28) et l'extrémité libre de la nervure (26).

2. Dispositif (10) selon la revendication 1, **caractérisé en ce que** le corps (12) comporte une face externe (20) recouverte par une couche (38) en un matériau bactéricide ou au moins bactériostatique et **en ce que** la couche de matériau (38) comporte une ouverture centrale (40) traversée par l'obturateur à fente (18) de manière telle que la face externe (20) de l'obturateur à fente (18) affleure avec la face externe (20) de la couche de matériau (38).

3. Dispositif (10) selon la revendication précédente, **caractérisé en ce que** la couche de matériau (38) est réalisée par dépôt métallique sur la face externe (36) du corps (12).

4. Dispositif (10) selon la revendication 2, **caractérisé en ce que** la couche de matériau (38) consiste en une plaque métallique sertie sur le corps (12), en recouvrant la face externe (36) du corps (12).

5. Dispositif (10) selon l'une quelconque des revendications 2 à 4, **caractérisé en ce que** la couche de matériau (38) est réalisée à partir de cuivre ou d'argent.

6. Dispositif (10) selon l'une quelconque des revendications 2 à 5 **caractérisé en ce que** la face avant (38a) de la couche de matériau (38) et la face externe (20) de l'obturateur à fente (18) forment une surface d'application plane, concave ou convexe.

7. Dispositif (10) selon l'une quelconque des revendications précédentes, **caractérisé en ce qu'**il est réalisé d'une seule pièce avec un récipient (16) contenant le produit.

8. Dispositif (10) selon la revendication 6 ou 7, **caractérisé en ce que** le récipient est rigide et comporte une paroi mobile (42) et une paroi fixe qui porte le dispositif (10).

9. Dispositif (10) selon la revendication 6 ou 7, **caractérisé en ce que** le récipient est rigide et comporte une paroi mobile (42) qui porte le dispositif (10).

## Patentansprüche

1. Vorrichtung (10) zum Abgeben eines fluidförmigen Mediums, enthaltend einen Körper (12), der sich in einer Ebene orthogonal zu einer Mittelhauptebene (A) der Vorrichtung (10) erstreckt und eine mittige Aufnahmeausnehmung (14) enthält,
einen Schlitzverschluss (18), der in der mittigen Aufnahmeausnehmung (14) des Körpers (12) angeordnet ist, der aus einem elastisch verformbaren Material ausgebildet ist und von einer Außenseite (20), einer Innenseite (22) und einer Umfangswand (24) begrenzt wird,
wobei der Schlitzverschluss (18) einen Schlitz (30) aufweist, der im Wesentlichen parallel zu der Mittelhauptebene (A) der Vorrichtung (10) verläuft, der sich von der Innenseite (22) zur Außenseite (20) erstreckt und der zwei Wände (30a, 30b) aufweist, die einander gegenüberliegen und in Anlage aneinander sind,
wobei der Schlitzverschluss (18) eine in der Innenseite (22) ausgebildete Rinne (32) aufweist, die zwei geneigte Flächen (34) aufweist, von denen jede eine Wand (30a, 30b) des Schlitzes (30) nach innen verlängert,
**dadurch gekennzeichnet, dass** die beiden gegenüberliegenden Wände (30a, 30b) dazu geeignet sind, sich durch Verlagerung senkrecht zu der Mittelhauptebene (A) der Vorrichtung (10) unter der Wirkung des Drucks des auszugebenden Mediums durch elastische Verformung des Schlitzverschlusses (18) zu beabstanden,
und dass der Schlitzverschluss (18) eine von seiner Umfangswand (24) vorstehende Rippe (26) aufweist, die in einer zugeordneten Nut (28) der mittigen Aufnahmeausnehmung (14) aufgenommen ist, und dass zwischen dem Boden der Nut (28) und dem freien Ende der Rippe (26) ein radiales Spiel vorliegt.

2. Vorrichtung (10) nach Anspruch 1, **dadurch gekennzeichnet, dass** der Körper (12) eine Außenseite (20) enthält, die von einer Schicht (38) aus einem bakteriziden oder zumindest bakteriostatischen Material bedeckt ist und dass die Materialschicht (38) eine mittige Öffnung (40) aufweist, die von dem Schlitzverschluss (18) so durchsetzt wird, dass die Außenseite des Schlitzverschlusses (18) mit der Außenseite (20) der Materialschicht (38) bündig liegt.

3. Vorrichtung (10) nach dem vorangehenden Anspruch, **dadurch gekennzeichnet, dass** die Materialschicht (38) durch Metallabscheidung auf die Außenseite (36) des Körpers (12) ausgebildet ist.

4. Vorrichtung (10) nach Anspruch 2, **dadurch gekennzeichnet, dass** die Materialschicht (38) aus einer Metallplatte besteht, die auf den Körper (12) aufgepresst ist und dabei die Außenseite (36) des Körpers (12) bedeckt.

5. Vorrichtung (10) nach einem der Ansprüche 2 bis 4, **dadurch gekennzeichnet, dass** die Materialschicht (38) aus Kupfer oder Silber gebildet ist.

6. Vorrichtung (10) nach einem der Ansprüche 2 bis 5, **dadurch gekennzeichnet, dass** die Vorderseite (38a) der Materialschicht (38) und die Außenseite (20) des Schlitzverschlusses (18) eine ebene, konkave oder konvexe Andrückfläche bilden.

7. Vorrichtung (10) nach einem der vorangehenden Ansprüche, **dadurch gekennzeichnet, dass** sie aus einem Stück mit einem das Medium enthaltenden Behälter (16) ausgebildet ist.

8. Vorrichtung (10) nach Anspruch 6 oder 7, **dadurch gekennzeichnet, dass** der Behälter starr ist und eine bewegliche Wand (42) und eine feste Wand enthält, die die Vorrichtung (10) trägt.

9. Vorrichtung (10) nach Anspruch 6 oder 7, **dadurch gekennzeichnet, dass** der Behälter starr ist und eine bewegliche Wand (42) enthält, die die Vorrichtung trägt.

## Claims

1. Device (10) for dispensing a fluid product, comprising a body (12) that extends in a plane orthogonal to a principal median plane (A) of the device (10), that comprises a central housing (14),
a slit stopper (18) arranged in the central housing (14) of the body (12), that is made from an elastically deformable material and that is delimited by an external face (20), an internal face (22) and a peripheral wall (24),
the slit stopper (18) comprising a slit (30) approximately parallel to the principal median plane (A) of the device (10), that extends from the internal face (22) to the external face (20), and that comprises two facing walls (30a, 30b) that bear in contact with each other,
the slit stopper (18) comprising a groove (32) made in the internal face (22), that comprises two inclined faces (34) each of which extends a wall (30a, 30b) of the slit (30) in the inwards direction,
**characterised in that** the two facing walls (30a, 30b) can move apart following a movement perpendicular to the principal median plane (A) of the device (10) under pressure from the product to be dispensed, by elastic deformation of the slit stopper (18),
and **in that** the slit stopper (18) comprises a rib (26) projecting from its peripheral wall (24) that fits into an associated groove (28) in the central housing (14), and **in that** there is a radial clearance present between the bottom of the groove (28) and the free end of the rib (26).

2. Device (10) according to claim 1, **characterised in that** the body (12) comprises an external face (20) covered by a layer (38) made of a bactericide or at least bacteriostatic material, and **in that** the material layer (38) comprises a central opening (40) through which the slit stopper (18) passes such that the external face (20) of the slit stopper (18) is flush with the external face (20) of the material layer (38).

3. Device (10) according to the previous claim, **characterised in that** the material layer (38) is made by metallic deposition on the external face (36) of the body (12).

4. Device (10) according to claim 3, **characterised in that** the material layer (38) consists of a metal plate crimped onto the body (12), covering the external face (36) of the body (12).

5. Device (10) according to any one of claims 3 to 5, **characterised in that** the material layer (38) is made from copper or silver.

6. Device (10) according to any one of claims 3 to 6, **characterised in that** the front face (38a) of the material layer (38) and the external face (20) of the slit stopper (18) form a plane, concave or convex application surface.

7. Device (10) according to any one of the previous claims, **characterised in that** it is made from a single part with a receptacle (16) containing the product.

8. Device (10) according to either claim 7 or 8, **characterised in that** the receptacle is rigid and comprises a moving wall (42) and a fixed wall on which the device (10) is fixed.

9. Device (10) according to either claim 7 or 8, **characterised in that** the receptacle is rigid and comprises a moving wall (42) on which the device (10) is fixed.
